Europäisches Patentamt

European Patent Office

Office européen des brevets

(19)

(11) Veröffentlichungsnummer: **0 246 520**
**B1**

(12) # EUROPÄISCHE PATENTSCHRIFT

(45) Veröffentlichungstag der Patentschrift: **12.12.90**

(51) Int. Cl.⁵: **A 61 F 5/41**

(21) Anmeldenummer: **87106740.1**

(22) Anmeldetag: **08.05.87**

(54) Erektionshilfe.

(30) Priorität: **21.05.86 DE 3617027**

(43) Veröffentlichungstag der Anmeldung:
**25.11.87 Patentblatt 87/48**

(45) Bekanntmachung des Hinweises auf die
Patenterteilung:
**12.12.90 Patentblatt 90/50**

(84) Benannte Vertragsstaaten:
**AT BE CH DE ES FR GB GR IT LI LU NL SE**

(56) Entgegenhaltungen:
**DE-C- 427 488**
**US-A-3 495 589**
**US-A-3 820 533**

(73) Patentinhaber: **Seeberg-Elverfeldt, Herbert, Dr.**
**Curtiusstrasse 22**
**D-4006 Erkrath 2 (DE)**

(72) Erfinder: **Seeberg-Elverfeldt, Herbert, Dr.**
**Curtiusstrasse 22**
**D-4006 Erkrath 2 (DE)**

(74) Vertreter: **Schaefer, Konrad**
**Gehölzweg 20**
**D-2000 Hamburg 70 (DE)**

**Beschreibung**

Die Erfindung betrifft eine Erektionshilfe der im Oberbegriff des Anspruchs 1 genannten Art.

Eine derartige Erektionshilfe ist aus der DE—C—427 488 bekannt. Es handelt sich dabei um einen elastischen aufblasbaren Ring, der den Penis elastisch umgreift und beim Aufblasen mittels des dazu vorgesehenen Ventiles den Penis einer Ringkompression unterwirft. Venöses, rückströmendes Blut wird dadurch gestaut, wobei das artiele zuströmende Blut nicht behindert wird. Die Schwellkörper werden gefüllt, und es stellt sich die gewünschte Erektion ein. Impotenz jeder Art, ob psychisch oder organisch bedingt, kann mit einer Erektionshilfe dieser Art behandelt werden.

Vorteilhaft bei dieser bekannten Konstruktion ist die Ausbildung der Erektionshilfe als weicher elastischer Schlauch, durch den jede Verletzungsgefahr vermieden wird. Es kann also weder der Penis verletzt werden, noch kann der Partner verletzt werden. Beim Anstoßen des Schlauches gegen den weiblichen Genitalbereich kann der weiche elastische Schlauch keinerlei Verletzungen hervorrufen.

Die bekannte Konstruktion ist als homogener Ringschlauch ausgebildet. Dieser Ringschlauch soll sich bestimmungsgemäß nach innen um ein bestimmtes Maß ausdehnen, um den gewünschten Kompressionseffekt zu erzielen. Von innen wird jedoch durch den Penis ein Widerstand erzeugt, der der Ausdehnung des Ringes nach innen entgegensteht. Der bekannte Ringschlauch dehnt sich daher überwiegend zur Seite aus, also in Richtung der Penislängsachse bzw. in Richtung der Symmetrieachse des Ringes. Es ergibt sich also in Richtung der Penislänge eine erhebliche Verbreiterung des Ringes. Dadurch wird in nachteiliger Weise die für den bestimmungsgemäßen Erfolg wesentliche freie Penislänge eingeschränkt bzw. sogar der Erfolg in Frage gestellt, wenn entsprechende physische Konstitution des Patienten hinzukommt, wie insbesondere Fettleibigkeit bei gleichzeitig vorliegender unterdurchschnittlicher Penislänge.

Die Aufgabe der vorliegenden Erfindung besteht daher darin, eine Erektionshilfe der im Oberbegriff des Anspruchs 1 genannten Art zu schaffen, die weich und elastisch ausgebildet ist, um Verletzungen zu verhindern und die so kurz wie möglich ausgebildet ist, um eine möglichst große nutzbare Penislänge zur Verfügung zu stellen.

Diese Aufgabe wird erfindungsgemäß mit den Merkmalen des Kennzeichnungsteiles des Anspruchs 1 gelöst.

Aus der US—A—3.495.589 ist eine Erektionshilfe mit einem elastischen, aufblasbaren Ring von durchweg gleicher Wandstärke bekannt, der längs seinem äußeren Umfang und seitlich von einem Gehäuse umgeben ist. Durch dieses Gehäuse wird bewirkt, daß der aufblasbare Ring sich einer nach innen, nicht aber zur Seite und nach außen ausdehnen kann.

Die erfindungsgemäße Erektionshilfe weist einen Ringschlauch auf, der auf seiner Außenseite formsteifer ist und beim Aufblasen des Schlauches eine Ausdehnung in Richtung der Symmetrieachse des Ringes, also in die Breite, verhindert. Der Ring kann sich also im wesentlichen nur nach innen in die gewünschte Richtung ausdehnen, behält beim Aufblasvorgang aber seine Breite im wesentlichen bei. Die nutzbare freie Penislänge wird also nicht unnötig eingeschränkt.

In vielen Fällen kann nur mit dieser Konstruktion der Erfolg sichergestellt werden.

Weitere vorteilhafte Ausgestaltungen der Erfindung ergeben sich aus den Unteransprüchen.

In den Zeichnungen ist die Erfindung beispielsweise und schematisch dargestellt. Es zeigen:

Figur 1: eine Ansicht des aufgeblasenen Schlauches in vergrößerter Darstellung,

Figur 2: einen Schnitt nach Linie 2—2 in Figur 1 und

Figur 3: einen Schnitt gemäß Figur 2, jedoch bei erhöhtem Aufblasdruck.

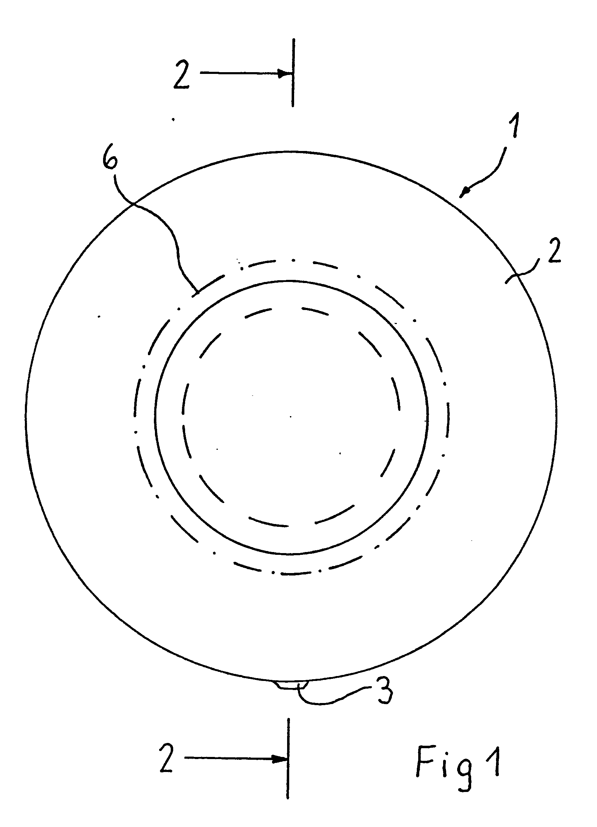

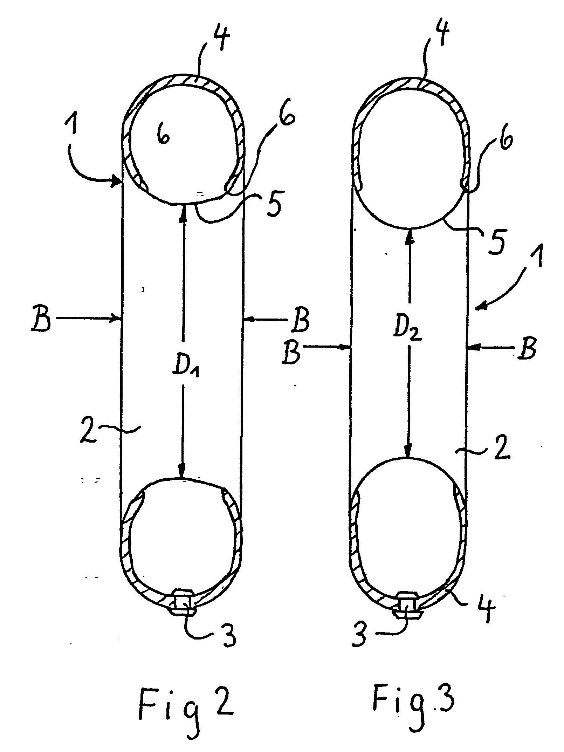

Wie Figur 1 zeigt, besteht die erfindungsgemäße Erektionshilfe aus einem Ring 1, der aus einem Schlauch 2 gebildet ist, dessen Querschnitt aus der Schnittdarstellung der Figur 2 ersichtlich ist.

An einer Stelle des Ringes ist im Schlauch ein der zeichnerischen Vereinfachung halber nur schematisch angedeutes Ventil 3 vorgesehen.

Es handelt sich um ein handelsübliches Ventil, das als Rückschlagventil mit Ablaßmöglichkeit ausgebildet ist. Mit einer einfachen Handpumpe, die am Ventil 3 außen ansetzbar ist, kann der Druck im Schlauch 2 erhöht werden. Durch geeignete Ventilbetätigung kann erhöhter Druck reduziert werden. Auf diese Weise kann der Innendruck des Schlauches 2 auf beliebige Weise variiert werden.

In der Darstellung der Figuren 1 und 2 ist der Ring nur schwach aufgeblasen, so daß er die benötigte Formstabilität erreicht, die zu seiner Handhabung nötig ist. In dieser Grundform besitzt er einen Schlauchdurchmesser (Ringbreite) B (siehe Figur 2) von etwa 7—10 mm. Der Innendurchmesser $D_1$ des Ringes beträgt in der Grundstellung ca. 25 mm in Auführung für Standardpenisabmessungen. Bei physiologischen Abweichungen können Unter- bzw. Übergrößen gefertigt werden.

In diesen Abmessungen insbesondere des Innendurchmessers D entspricht der Ring dem normalen Penisdurchmesser und läßt sich mit leichtem Spiel über diesen schieben. Schon bei geringer Verringerung des Innendurchmessers durch Aufblasen des Ringes wird Druck auf den Penis in der gewünschten Weise ausgeübt.

Bestünde der Schlauch gemäß dem Stand der Technik aus Material allseitig gleicher Wandstärke, so würde beim Aufblasen des Ringes nicht nur der Innendurchmesser $D_1$ abnehmen, sondern es würde auch der Außendurchmesser wachsen und insbesondere würde in störender Weise die Breite B anwachsen. Da der Verringe-

rung des Innendurchmessers $D_1$ der Gegendruck des Penis entgegensteht, würde insbesondere die Breite B stärker anwachsen. Es würde sich bei Erzeugen des gewünschten Druckes unter Umständen mehr als die dreifache Breite ergeben. Die freie Penislänge würde dadurch um bis über 20 mm störend eingeschränkt, was unter Umständen den Erfolg völlig in Frage stellt.

Erfindungsgemäß ist daher der in bezug auf die Symmetrieachse des Ringes 1 außen liegende Teil des Schlauches 2 verstärkt ausgebildet. Figur 2 zeigt dies im Schnitt durch den Schlauch 2. Der außen liegende Bereich 4 des Schlauches ist verstärkt ausgebildet gegenüber dem dünnen innenliegenden Bereich 5. Der Übergang erfolgt vorteilhaft in der dargestellten Weise allmählich, so daß keine störenden Kanten entstehen.

In den Figuren ist eine bevorzugte Ausführungsform dargestellt. Figur 2 zeigt einen Schnitt durch die Symmetrieachse des Ringes. Dort ist zu erkennen, daß im Schlauchquerschnitt etwa dreiviertel des Umfanges des Schlauches aus Material größerer Wandstärke besteht. Der innenliegende, also zur Symmetrieachse des Ringes hin gelegene kleinere Bereich 5 besteht aus Material dünnerer Wandstärke.

Wird diese erfindungsgemäße Konstruktion durch Aufblasen am Ventil 3 auf höheren Innendruck gebracht, wie dies Figur 3 zeigt, so dehnt sich der dünnwandige innen liegende Bereich 5 nach innen aus, und es ergibt sich die gewünschte Verringerung des Innendurchmessers auf $D_2$.

Figur 1 zeigt mit gestrichelter Linie diesen geringeren Innendurchmesser.

Da der außen liegende verstärkte Bereich 4 bei dieser geringen Druckerhöhung seine Form nur wenig oder gar nicht verändert, bleibt der Außendurchmesser im wesentlichen erhalten. Im Sinne der erfindungsgemäßen Funktion steht aber die Tatsache im Vordergrund, daß sich die Breite B nicht oder nur sehr geringfügig ändert. Der Querschnitt des Schlauches 2 ändert sich also, wie ein Vergleich der Figuren 2 und 3 zeigt, von rundem Querschnitt gemäß Figur 2 zu ovalem Querschnitt gemäß Figur 3.

Figur 2 zeigt die Aufblasstellung mit dem größeren Innendurchmesser $D_1$, in welcher der Ring auf dem Penis appliziert wird. Der Innendurchmesser $D_1$ soll also im wesentlichen dem Penisdurchmesser entsprechen. Der Ring kann aber auch so ausgebildet sein, daß er nicht in seinem Innendurchmesser $D_1$, sondern im Durchmesser der Linie 6 dem Penisdurchmesser entspricht. Er ist dann mit leichter Kraft unter Aufweitung des elastischen Teiles 5 aufzuschieben. Von Vorteil ist dabei, daß der versteifte Bereich 4 mit seinem Rand 6 unmittelbar bis zum Penis reicht, seitliche Verformungen des Ringes also völlig ausgeschlossen sind.

## Patentansprüche

1. Erektionshilfe, bestehend aus einem ringförmigen, dünnwandigen Schlauch (2) aus elastischem und weichem Material mit einem Aufblasventil (3), dadurch gekennzeichnet, daß der Schlauch (2) im Schnitt durch die Ringachse einen innenliegenden, in seiner Umfangserstreckung kleineren Bereich (5) geringerer Wandstärke und einen außen liegenden, in seiner Umfangserstreckung größeren Bereich (4) größerer Wandstärke aufweist so daß der außenliegende größere Bereich (4) in Bezug auf den innenliegenden Bereich (5) versteift ausgebildet ist.

2. Erektionshilfe nach Anspruch 1, dadurch gekennzeichnet, daß der innen liegende Bereich (5) etwa ein Vierteil des Querschnittsumfanges beträgt.

3. Erektionshilfe nach Anspruch 1, dadurch gekennzeichnet, daß der Innendurchmesser ($D_1$) des Ringes (1) dem Penisdurchmesser entspricht.

## Revendications

1. Auxiliaire d'érection, comprenant un tuyau annulaire (2) à paroi mince en un matériau élastique et souple, avec une valve de gonflage (3), caractérisé en ce que ce tuyau (2) présente à la section par l'axe de l'anneau formé une zone intérieure d'extension (5) périphérique moindre dans laquelle la paroi est plus mince et une zone extérieure (4) plus grande dans laquelle la paroi est plus épaisse de sorte que la zone extérieure (4) soit plus raide que la zone intérieure (5).

2. Auxiliaire d'érection selon la revendication 1, caractérisé en ce que la zone intérieure (5) s'étend sur environ un quart de la périphérie de la section transversale.

3. Auxiliaire d'érection selon la revendication 1, caractérisé en ce que le diamètre intérieur ($D_1$) de l'anneau (1) correspond au diamère du pénis.

## Claims

1. Erection aid consisting of an annular, thin-walled tube (2) of an elastic and soft material with an inflation valve (3), characterized by the tube (2) in the section through the ring axis having an internal, in its peripheral extend smaller range (5) of lesser wall thickness and an external, in its peripheral extend larger range (4) of larger wall thickness so that the external larger range (4) is stiffer with respect to the internal range (5).

2. Erection aid according to Claim 1, characterized by the internal range (5) being approximately one quarter of the cross-sectional periphery.

3. Erection aid according to claim 1, characterized by the inner diameter ($D_1$) of the ring corresponding with the diameter of the penis.

Fig 1

Fig 2          Fig.3